Europäisches Patentamt

European Patent Office

Office européen des brevets

(19)

(11) Publication number: **0 286 434**
**A2**

# (12) EUROPEAN PATENT APPLICATION

(21) Application number: **88303173.4**

(22) Date of filing: **08.04.88**

(51) Int. Cl.⁴: **C 12 Q 1/04**

(30) Priority: **08.04.87 US 35764**

(43) Date of publication of application:
**12.10.88 Bulletin 88/41**

(84) Designated Contracting States:
**AT BE CH DE ES FR GB GR IT LI LU NL SE**

(71) Applicant: **CAMBRIDGE BIOSCIENCE CORPORATION**
**365 Plantation Street**
**Worcester Massachusetts 01605 (US)**

(72) Inventor: **Waskiewicz,David E.**
**15 Mechanic Street**
**Milford, Massachusetts 01757 (US)**

**Patel,Usha**
**16-11 Shadowbrook Lane**
**Milford, Massachusetts 01757 (US)**

**Marcian,Dante J.**
**48 School Street**
**Hopkinton, Massachusetts 01748 (US)**

(74) Representative: **Sheard, Andrew Gregory et al**
**Kilburn & Strode 30, John Street**
**London WC1N 2DD (GB)**

(54) Method for the detection of bacteria and fungi.

(57) A method for detecting bacteria and fungi in a sample by enzymatic activity of the bacteria and fungi is disclosed. The suspected bacteria or fungi in the sample are first lysed with a combination of lytic enzyme and detergent to rupture the bacterial and fungal cell walls. The bacteria and fungi are then detected via the enzymatic activity of the bacteria and fungi by an enzyme detection assay.

EP 0 286 434 A2

**Description**

## METHOD FOR THE DETECTION OF BACTERIA AND FUNGI

The present invention relates to the detection of bacteria and fungi in a sample, especially in aqueous physiological fluids. More specifically, this invention relates to a method for lysing the bacterial and fungal cell walls using a combination of a lytic enzyme and detergent and determining the bacterial and fungal enzyme activity with an enzyme detection system.

Among the most frequently occurring bacterial and fungal infections are those of the urinary tract. Many different microorganisms can infect the urinary tract. These organisms include gram-negative bacteria such as Escherichia coli, Proteus sp., Klebsiella sp., and Pseudomonas sp., gram-positive bacteria such as Staphylococcus sp. and Enterococcus sp., and fungi such as the yeast Candida.

These infections are commonly confirmed in clinical laboratories by time-consuming procedures which involve culturing of specimens.

In general, the significance of quantitative microbiological examination of urine is frequently complicated by sample contamination. Colony counts of 10,000 ($10^4$) to 100,000 ($10^5$) or more organisms per milliliter are considered indicative of infection when urine samples are obtained by the clean-voided technique or by catheterization. Colony counts of less than 10,000 ($10^4$) per milliliter usually reflect contamination. Aseptic sampling methods are often required to associate a reasonable statistical probability of bacteriuria when the colony counts are below $10^5$ per milliliter. In addition, quantitative results require interpretation in terms of a patient's symptoms, the time of day, urine sampling methodology, quantitation methodology, and administration of therapeutic medication, if any.

A variety of culture-based methods are utilized for quantitating bacteriuria. The pour plate dilution technique is the most precise, but requires 24 to 48 hours for a determination. Other quantiative methods use a standardized calibrated inoculating loop and streak plate. Semi-quantitative methods include the dip-slide, filter paper, pipette, cup and pad culture. All of these methods require the growth of bacteria on media. Chemical methods are based on the Griess test for nitrate, triphenyl tetrazolium reduction, glucose oxidase reduction with glucose and catalyst-hydrogen peroxide interaction. These methods are not, however, as reliable, sensitive or specific as the culture test. A number of instrumented procedures are also available, but these require relatively costly hardware and experienced technicians.

Hence, there is a continuing need for a simple, reliable and inexpensive procedure for rapid detection of bacteria which avoid the short comings of known systems.

The present invention provides for a method of detecting bacteria or fungi in a sample by enzymatic activity of the bacteria or fungi. The suspected microorganisms in a sample are first lysed with a combination of lytic enzyme and detergent to rupture the cell walls. The bacteria or fungi are then detected via the enzymatic activity of the microorganism by an enzyme detection assay.

The inventors have found that known bacterial or fungal enzyme detection assays are enhanced by the use of the combination of detergents and lytic enzymes to lyse the cell walls of the microorganisms. Thus, the detection signal is significantly increased by treating the microorganism according to the method of this invention.

The present invention relates to a method for the detection of bacteria or fungi in a sample. The sample is lysed with a combination of lytic enzymes(s) and detergent(s) which ruptures the microbial cell walls. The enzymatic activity of the lysed bacteria or fungi is then capable of being detected by an enzyme assay.

The sample suspected of having bacteria or fungi may include food, tissue, ground water, cooling water, pharmaceutical products, sewage, etc. The invention is particularly useful for detection of bacteria in aqueous liquids, such as human and animal biological fluids, such as urine, spinal fluid, blood, and the like as well as stool secretions and suspensions of human or animal tissue. The preferred biological fluid used in practising this invention is urine.

In the practice of this invention it is preferable that the sample is first treated to remove large or excess contaminating debris by centrifugation or filtration. This debris may mask, compete, or otherwise interfere with the microbial lysis or the enzyme detection system. For example, the sample may be passed through a filter in order to separate the bacterial cells from other cellular debris present in the sample. The filtrate will then comprise primarily the bacteria to be detected and other unicellular organisms. It may then be preferable to concentrate the bacteria by passing the filtrate through a second, finer filter which can trap the bacteria on a small surface area. Bacteria are detected by applying the lytic enzyme, detergent and enzyme detection assay components to this second filter.

Lytic enzymes are enzymes that can break microbial cell walls by hydrolysis of cell wall components. Enzymes possessing lytic activity include autolysins, glycosidases, amidases, and endopeptidases. Among the specific lytic enzymes that may be used in this invention are lysozyme, mutanolysin, lysostaphin, and chitinase.

Detergents, as defined herein, are any of numerous synthetic water-soluble or liquid organic preparations that are, for example, able to emulsify oils, hold particles in suspension, and act as wetting agents. Detergents that may be used in this invention may be ionic, nonionic, or zwitter ionic, and may include, but are not limited to, the following: Tween-20 (polyoxyethylenesorbitan monolaurate), Tergitol NP-40 (a polyglycol ether surfactant), and Triton X-114 (octylphenoxypolyethoxyethanol). "Tergitol" is a registered trademark of Union Carbide Corporation. "Triton" is a registered trademark of the Rohm &; Haas Company.

The concentration of lytic enzyme may range from 10 ng/ml to 5 mg/ml, and the detergent concentration may range from 0.001% to 2%. Further, one or more lytic enzyme can be used with one or more detergents according to this invention.

In the first step in the method of detecting bacteria or fungi according to this invention, the sample suspected of containing the bacteria or fungi is contacted with a combination of lytic enzyme(s) and detergent(s) to rupture or lyse the microbial cell wall. In another embodiment of this invention, the lysis of the microbial cell wall may be performed simultaneously with the use of the enzyme detection assay. There may be enzyme detection assays known in the art which would be adversely affected by the simultaneous lysing and detection, and therefore, the lysing step and enzyme detection step should for preference be done sequentially.

The enzyme detection assays employed in the practice of the present invention are known in the art and many of them are commercially available. In a preferred embodiment of this invention, the assay used detects the dehydrogenase activity of lysed bacteria. In this system the dehydrogenase activity of the bacteria is coupled to a cationic dye, nitro blue tetrazolium (NBT), a color indicator. Coupling is carried out via an intermediate carrier, Meldola blue (8-dimethylamino-2, 3-benzophenoxazine), an electron carrier. Other electron carriers that may be used, include resorufin, phenazine methosulfate, and phenazine ethosulfate. NBT is particularly useful in this invention because it is converted on reduction from a light yellow, almost colorless, water soluble compound to a deep blue precipitate. This blue reduced form remains at the reduction site as insoluble formazan. Excess reagent does not affect the assay sensitivity because the reagent cannot dilute the insoluble reaction product.

Another enzyme detection system that may be used in this invention is the 2,6-dichloroindophenol assay system. This assay system also detects dehydrogenase activity and requires the use of one or more electron carriers e.g., phenazine ethosulfate. Upon reduction, 2, 6-dichloroindophenol is converted from blue to colorless.

In general, the bacterial or fungal detection limit will depend on the microbial concentration in the sample, the method of concentrating the microorganisms (if any), the species and metabolic state of the microorganism(s), and the sensitivity of the enzyme detection system. For example, bacteria can be detected in urine at the level of 10,000 ($10^4$) per mL with the nitro blue tetrazolium assay when the bacteria from a 1 mL sample are collected on a 3mm diameter filter disk.

The materials for use in the method of this invention are suited for the preparation of a kit. Such a kit may comprise a carrier means being compartmentalized to receive in close confinement one or more container means, such as vials, tubes, and the like. Each of the container means comprises one of the separate elements that are used in the method of this invention. For example, one of the container means may contain the material for concentrating the microorganisms, such as filter or semi-permeable membranes to retain bacterial or fungi. The second container may contain the combination of the lytic enzyme and detergent. A third or fourth container means may contain the various components of the enzyme detection assay.

In addition, the carrier means may also contain a plurality of container means each of which contains a different, predetermined amount of known bacterial or fungal enzyme to use as a standard. These containers can then be used to prepare a standard curve to compare against the results obtained by using the method of this invention to detect the presence of bacteria in a sample.

In using the kit, a user adds to a container a premeasured amount of a sample suspected of containing the bacteria or fungi and concentrates the sample and/or removes interfering substances, if needed. The user then adds the contents of the first container containing the combination of the lytic enzyme and detergent, and the contents of the second or third container containing the enzyme detection assay. After an appropriate time for colour development, according to the particular enzyme detection system used, the lysed bacteria or fungi are detected by measuring the enzymatic activity.

It should be understood that not every detergent or lytic enzyme combination will enhance every enzyme detection assay for every microbial species. The determination of whether a given lytic enzyme and detergent combination and enzyme detection assay is operable and within the scope of the present invention can be made by a simple test. This test comprises adding the lytic enzyme and detergent to a microbial sample and running a control sample simultaneously and observing whether the enzyme detection assay is enhanced by the combination of the lytic enzyme and detergent. Generally, an increase in the intensity of the assay's detection signal is an indication of enhancement of the enzyme detection system.

The following examples are intended to illustrate, but not limit, the method of the present invention.

## EXAMPLE 1

### THE TEST SYSTEM

In examples 2-6, bacteria were detected using the following general procedure.

Bacteria were clinical strains obtained from the Newport (Rhode Island) Naval Hospital and cultured on nutrient agar plants (Scott Laboratories, Fiskeville, Rhode Island). Samples for testing were suspensions of bacteria, usually at a concentration of $10^7$ to $10^8$ CFU (colony forming unit) per mL, in phosphate buffered saline. Bacterial concentrations were calculated assuming an absorbance of 0.400 at 650nm for a suspension of $1 \times 10^8$ CFU per mL and were confirmed by plating on culture plates. These suspensions were kept at 2-8°C until use.

The assay solution was prepared freshly in water and contained the following: 0.17 mg/ml nitro blue tetrazolium (NBT) (Calbiochem, San Diego, California), 0.311 ug/ml Meldola blue (Boehringer Mannheim Biochemicals, Indianapolis, Indiana), 0.096 mg/ml L-lactic acid (Sigma Chemical Co., St. Louis, Missouri), 1.35 mg/ml L-malic acid (Sigma), 0.130 mg/ml glucose (Fisher Scientific, Pittsburg, PA), 0.56 mg/ml sodium phosphate monobasic monohydrate (Fisher), 4.42 mg/ml beta-nicotinamide adenine dinucleotide (Boehringer Mannheim), and 0.3 M 2-amino-2 (hydroxymethyl)-1,3-propandiol (Sigma). Lytic enzymes and/or detergents (all from Sigma) were added to the levels indicated. The pH of the solution was adjusted to 9.0, and the solution was kept at 2-8 C until use.

The assays were performed in 96-well microtiter plates (Dynatech Laboratories, Alexandria, Virginia) by mixing 80 ul of assay solution and 20 ul of sample, and incubating for 30 minutes at 37°C. The absorbances of the wells were determined in a Dynatech MR-600 Microplate Reader at 570 nm, and ranged from 0.05 to 1.00 for positive samples. These absorbances were corrected for background by subtracting the absorbances of negative controls in which 20 ul phosphate buffered saline replaced the bacterial sample, and by subtracting the contribution of the bacteria alone.

EXAMPLE 2

Suspensions of six bacterial species were tested as described in Example 1. The effect of the addition of the lytic enzymes lysozyme (0.1 mg/ml) and the detergent Tween-20 (0.05%) on colour development was then determined. The results appear in Table I.

## TABLE I

### Relative Absorbance of NBT Color Development for Different Bacterial Detection Formulas
(Signal in the absence of lysozyme and Tween = 1.00)

#### Bacterial Species

| Assay Additives | Ec | K | Pr | En | S | Ps |
|---|---|---|---|---|---|---|
| With lysozyme + Tween-20 | 12.0 | 5.4 | 11.9 | 1.3 | 1.1 | 0.9 |
| With lysozyme alone | 1.2 | 1.4 | 1.4 | 1.0 | 0.9 | 1.0 |
| With Tween-20 alone | 2.1 | 1.0 | 3.3 | 1.3 | 1.3 | 1.1 |
| Without lysozyme or Tween-20 | 1.0 | 1.0 | 1.0 | 1.0 | 1.0 | 1.0 |

Ec = Escherichia coli
K = Klebsiella pneumoniae
Pr = Proteus mirabilis
En = Enterococcus (group D strep)
S = Staphylococcus epidermidis
Ps = Pseudomonas aeruginosa

EXAMPLE 3

In this example, two bacterial suspensions were assayed according to Example 1. The effect on the colour development by the addition of the lytic enzyme lysozyme (0.1 mg/ml) and of the two detergents, Tergitol-NP40 (0.05%) or Triton X-114 (0.05%) was determined with and without added lysozyme. The results appear in Table II. "Tergitol" is a registered trademark of Union Carbide Corporation. "Triton" is a registered trademark of the Rohm &; Haas Company.

## TABLE II

Relative Absorbance of NBT Color Development for Different Detergents (Signal in the absence of lysozyme and detergent = 1.0).

|                                              | Bacterial Species |      |
|----------------------------------------------|:-----:|:----:|
| Assay Additives                              | Ec    | Pr   |
| With lysozyme AND Tergitol-NP40 Tergitol-NP40 | 5.4   | 9.1  |
| With lysozyme alone                          | 1.1   | 1.1  |
| With Tergitol-NP40 alone                     | 1.4   | 2.6  |
| Without lysozyme or Tergitol NP40            | 1.0   | 1.0  |
| With lysozyme AND Triton X-114               | 5.8   | 9.2  |
| With lysozyme alone                          | 1.1   | 1.1  |
| With Triton X-114 alone                      | 3.6   | 7.5  |
| Without lysozyme or Triton X-114             | 1.0   | 1.0  |

Ec = Escherichia coli
Pr = Proteus mirabilis

EXAMPLE 4

In this example, a suspension of Pseudomonas aeruginosa was assayed using a modification of the procedure in Example 1. Bacteria in phosphate-buffered saline were pretreated with the lytic enzyme lysostaphin (0.5 mg/ml) and/or the detergent Tween-20 (0.05%) for 30 minutes at 37°C before the Assay Solution was added as described in Example 1. The effect on the color development of NBT by lysostaphin and Tween-20 was determined and are summarized below in Table III.

## TABLE III

Relative Absorbance of NBT Color Development for the Lytic Enzyme Lysostaphin (Signal in the absence of lysostaphin and Tween-20 = 1.0)

| Assay Additives                    | Pseudomonas aeruginosa |
|------------------------------------|:----:|
| With lysostaphin and Tween-20      | 2.20 |
| With lysostaphin alone             | 1.03 |
| With Tween-20 alone                | 1.06 |
| Without lysostaphin or Tween-20    | 1.00 |

EXAMPLE 5

In this example, an Escherichia coli suspension was assayed according to Example 1 and treated with a colour producing reagent containing tetranitro blue tetrazolium (Sigma) at 0.34 mg/ml in place of NBT. The effects on the color development by the addition of lysozyme 0.1 mg/ml and Tween-20 0.05% were then determined. The results appear in Table IV.

## TABLE IV

Relative Absorbance of Colour Development using Tetranitro Blue Tetrazolium (Signal in the absence of lysozyme and Tween 20 = 1.0).

| Assay Additives | Escherichia coli |
|---|---|
| With lysozyme and Tween-20 | 2.9 |
| With lysozyme alone | 1.2 |
| With Tween-20 alone | 1.5 |
| Without lysozyme or Tween-20 | 1.0 |

EXAMPLE 6

In this example, two bacterial suspensions were assayed using a modification of Example 1.

The assay solution used in this example was prepared freshly in water and contained 0.022 mg/ml 2,6-dichloroindophenol (Sigma), 0.096 mg/ml L-lactic acid (Sigma), 1.35 mg/ml L-malic acid (Sigma), 0.30 mg/ml glucose (Fisher), 4.42 mg/ml beta-nicotinamide adenine dinucleotide (Boehringer Mannheim), 0.076 mg/ml phenazine ethosulfate (Sigma), 10mM sodium phosphate (Fisher), and 0.15 M sodium chloride. The pH of the solution was adjusted to 7.5, and the solution was kept at 2-8°C until use. Samples were prepared as in Example 1.

The assays were performed in 96-well microtiter plates (Dynatech) by mixing 80 ul of assay solution and 20 ul of sample and incubating for 10 to 30 minutes at 37°C. The absorbance changes of the wells were determined in a Dynatech MR-600 Microplate Reader at 630 nm. Assays containing buffer only showed no changes in absorbance. Assays containing bacteria showed a decrease in absorbance. The effect on the absorbance changes by the addition of the lytic enzyme lysozyme (0.1 mg/ml) and the detergent Tween-20 (0.05%) are presented in Table V.

## TABLE V

Relative Absorbance Changes using 2,6-dichloro-indophenol. (Signal in the absence of lysozyme and Tween-20 = 1.0).

| Assay Additives | Escherichia coli | Proteus mirabilis |
|---|---|---|
| With lysozyme + Tween-20 | 4.3 | 6.8 |
| With lysozyme alone | 1.7 | 1.4 |
| With Tween-20 alone | 3.0 | 5.6 |
| Without lysozyme or Tween-20 | 1.0 | 1.0 |

Although the foregoing invention has been described in some detail by way of illustration and example for purposes of clarity and understanding, it will be obvious that certain changes and modifications may be practiced within the scope of the invention, as limited only by the scope of the appended claims.

## Claims

1. A method for detecting bacteria or fungi in a sample, comprising:
lysing a sample that is suspected of containing bacteria or fungi with a combination of lytic enzyme and detergent;
contacting the lysed sample with an enzyme detection system capable of detecting the bacterial or fungal enzyme activity; and
detecting the presence of any bacteria or fungi in the sample.

2. A method as claimed in claim 1, wherein the lysing with the lytic enzyme and detergent and the contacting with the enzyme detection system are done either (a) sequentially or (b) simultaneously.

3. A method as claimed in claim 1 or 2, wherein the sample is animal or human fluid or tissue.

4. A method as claimed in any of claims 1 to 3 wherein the lytic enzyme is an autolysin, glycosidase, amidase, or endopeptidase.

5. A method as claimed in any of claims 1 to 4, wherein the enzyme detection system comprises nitro blue tetrazolium and an electron carrier.

6. A method as claimed in any of claims 1 to 5 further comprising filtering the sample prior to lysing.

7. A method as claimed in claim 6 wherein the filtered sample is further filtered to concentrate the bacteria or fungi contained in the filtered sample.

8. A method as claimed in any of claims 1 to 5 further comprising filtering the sample to remove any interfering debris in the sample and to concentrate any bacteria or fungi in the sample.

9. A kit for detecting bacteria or fungi in a sample, comprising a carrier means being compartmentalised to receive in close confinement therein one or more containers wherein
(a) a first container contains a lytic enzyme and detergent; and
(b) a second container contains an enzyme detection system.

10. A kit as claimed in claim 9 further comprising a means of filtering the sample to remove any interfering debris from the sample and to concentrate any bacteria or fungi in the sample.